# EUROPEAN PATENT APPLICATION

(11) **EP 2 025 307 A1**
(43) Date of publication of application: **18.02.2009**
(21) Application number: 07744705.0
(22) Date of filing: 05.06.2007
(51) Int. Cl.: A61F 2/44

(54) **SPINOUS PROCESS SPACER**

(30) Priority: 07.06.2006 JP 2006158437
(71) Applicant: Olympus Terumo Biomaterials Corp., Shinjuku-ku Tokyo 163-0914 (JP)
(72) Inventor: SHIBATA, Koichi, Hino-shi Tokyo 191-0041 (JP); IRIE, Hiroyuki, Akishima-shi Tokyo 196-0024 (JP); URATA, Mitsuya, Kawasaki-shi Kanagawa 216-0005 (JP); OJIMA, Satoshi, Nishitokyo-shi Tokyo 202-0002 (JP)
(74) Representative: von Hellfeld, Axel
(86) International application number: PCT/JP2007/061350
(87) International publication number: WO 2007/142226

(57) **Abstract**

To provide a spinous process spacer that can be stably inserted and fixed between split faces of a split spinous process in a gapless manner, without made-to-order production for each patient. There is provided a spinous process spacer (1) for insertion between split halves of a spinous process resulting from longitudinal splitting of the spinous process, wherein the spinous process spacer is formed in an approximately cylindrical shape and the opposite end faces (2, 3) thereof are constituted by planes which mutually form a fixed angle.

## Description

### Technical Field

The present invention relates to a spinous process spacer for insertion between split halves of a spinous process resulting from longitudinal splitting of the cervical spinous process.

### Background Art

Conventionally, foraminotomy has been conducted for relieving impairments caused by compression on the spinal cord due to cervical spondylotic myelopathy or ossification of posterior longitudinal ligament. Double-door laminoplasty by midline splitting of spinous processes has been known in which spinous process spacers are inserted between split halves of spinous processes formed by longitudinal splitting of the spinous processes (for example, refer to Patent Document 1).
As a spinous process spacer to be used for this double-door laminoplasty, Patent Document 1 has disclosed a block-shaped spinous process spacer in which mutually orthogonal cross sections of two directions (horizontal cross section and longitudinal front forehead cross section) are both formed in approximately trapezoidal shapes.

The double-door laminoplasty is a method in which spinous processes are longitudinally split and thus formed interspaces between split halves of the spinous processes are pressingly opened to be inserted with spinous process spacers. The interspace formed by pressingly opening split halves of a split spinous process does not have parallel sides due to the balance of internal stress, since the spinous process itself has an irregular cross-sectional shape. Generally, the interspace is inwardly tapered and outwardly widened towards the spinal canal, and is widened towards the head side and tapered towards the coccygeal side.

Here, in the description of the present specification, for the sake of convenience, directions towards the inside or outside of the spinal canal are referred to as the horizontal direction and directions towards the head or coccyges along the spinal canal are referred to as the longitudinal direction under an assumption that the spinal canal is vertically upright.
The spinous process spacer of Patent Document 1 is intended to be suited into such an interspace extending towards two horizontal and longitudinal directions, and thereby employs a block-shape in which two orthogonal longitudinal cross sections are both formed in approximately trapezoidal shapes.
Patent Document 1:
The Publication of Japanese Patent No. 3660696

### Disclosure of Invention

However, as described above, the interspace between split halves of the spinous process is defined according to the balance of internal stress which is determined on the basis of the cross-sectional shapes of the spinous process and the like. Therefore the open angle thereof has individual difference. That is to say, although the horizontal open angle is set the same, the vertical open angle varies depending on the patient. Accordingly, even if such a spinous process spacer having approximately trapezoidal longitudinal cross sections respectively in two orthogonal directions as shown in Patent Document 1 is employed, not the same spinous process spacer is always suitable for every patient.

If only one type of spinous process spacer is applied to every patient, there is a concern in that the open angle of the interspace between split halves of the spinous process becomes so large depending on the patient that wedge-shaped gaps are formed respectively between the spinous process spacer and two split faces of the split spinous process. In such a case, the split faces of the spinous process and the spinous process spacer are in contact with each other in extremely small contact areas, and their contact condition is unstable, which may cause postoperative displacement with passage of time. Moreover, the trapezoid-shaped spacer has a possibility in which, when the neck is extended, vertically inserted spacers are rotated to collide with each other or interfere with the spinal cord.

The present invention takes the above problems into consideration with an object of providing a spinous process spacer that can be stably inserted and fixed between split faces of a split spinous process in a gapless manner, without made-to-order production for each patient.

In order to achieve the above object, the present invention provides the following solutions.
That is to say, one aspect of the present invention is a spinous process spacer for insertion between split halves of a spinous process resulting from longitudinal splitting of the spinous process, wherein the spinous process spacer is formed in an approximately cylindrical shape and the opposite end faces thereof are constituted by planes which mutually form a fixed angle.

For inserting the spinous process spacer between split halves of the spinous process by midline splitting of the spinous processes, when the interspace between split halves of a spinous process is opened, split faces of the split spinous process are horizontally inclined to be tapered towards the spinal canal side, and are vertically inclined to be widened towards the head side. Therefore, the horizontal open angle and the vertical open angle formed by the split faces of the split spinous process vary depending on the patient.

However, these two split faces of the split spinous process per se are retained in approximately planer shapes. Therefore, regardless of how the vertical and horizontal open angles are changed, these split faces are still two planes intersecting each other in one straight line (hereinunder, referred to as a cross line) at any location on their respective extensions. In this case, the angle formed by these two split faces is defined as an interior angle of a plane orthogonal to the above cross line (hereinunder, referred to as a reference plane).

Assuming that the split faces have only a horizontal open angle without being opened in the vertical direction, the above cross line is retained in the vertical direction. However, when the split faces are also opened in the vertical direction, the cross line is inclined so that the reference plane orthogonal to the cross line also rotates by the same angle. Then, the angle formed by the two split faces due to this rotation increases as they are more vertically opened.

The spinous process spacer according to the above aspect is formed in an approximately cylindrical shape, and the opposite end faces thereof are constituted by planes which mutually form a fixed angle. Therefore, by rotating the spinous process spacer about its central axis, the inclination directions of the opposite end faces can be aligned with the inclination directions of the split faces. Moreover, by narrowing the horizontal opening by the amount incremented due to the vertical opening of the split faces, the angle formed by the split faces can be aligned with the angle formed by the opposite end faces of the spinous process spacer.

Accordingly, the end faces can be closely attached to the split faces of the split spinous process of every patient by adjusting the rotation angle about the central axis, and by adjusting the horizontal opening, according to the variation of the vertical open angle of the split faces due to individual difference of the patient.
As a result, the spinous process spacer according to the above aspect can be prepared without a need of made-to-order production for each patient, and the same spinous process spacer can be applied to every patient. Moreover, its shape can also be so extremely simplified that the production can be performed at considerably low cost.

Furthermore, the spinous process spacer according to the above aspect is formed in an approximately cylindrical shape. Therefore, even if the spacer is rotated about its central axis, there is no need of much changing of the position of the outer peripheral surface. Accordingly, the spinous process spacer can be kept from interfering with an adjacent cervical spinous process or a spinous process spacer fixed thereto.

The spinous process spacer according to the above aspect preferably has an approximately mirror symmetric shape about a plane which passes through the longitudinal center and is orthogonal to the central axis.
The two split split faces resulting from longitudinal splitting of a spinous process are constituted in an approximately symmetrical manner, and are inclined in an approximately symmetrical manner by opening the interspace therebetween. Accordingly, the end faces can be closely attached to both split faces of the split spinous process, by constituting the spinous process spacer in an approximately mirror symmetric shape about a plane which passes through the longitudinal center and is orthogonal to the central axis.

Moreover, in the above aspect, the outer peripheral surface is preferably provided with an engaging portion to be engaged with a tool for applying a rotational force about the axis.
As described above, by rotating the spacer about its central axis while the spacer is being inserted into the interspace between the split faces of the split spinous process, the opposite end faces can be adjusted to be closely attached to the split faces. Accordingly, by engaging a tool with the engaging portion provided on the outer peripheral surface, the spinous process spacer can be readily rotated about its axis so that the abovementioned adjustment operation can be facilitated.

Furthermore, in the above structure, the engaging portion may also be formed of two mutually-parallel flat faces extending along the axis.
By so doing, the spinous process spacer can be readily rotated about its axis by setting a general-purpose tool such as a spanner so as to clip the spacer at these two flat faces provided as the engaging portion.

Moreover, in the above aspect, the opposite end faces may also be provided at an angle not smaller than 30° and not larger than 90°.
This is because that an angle within the above range is appropriate as an open angle between split faces of a split spinous process for enlarging the spinal canal.
Furthermore, the spinous process spacer according to the above aspect is preferably made of a biocompatible ceramic material.

The present invention demonstrates effects in which the spacer can be stably inserted and fixed between split faces of a split spinous process in a gapless manner, without made-to-order production for each patient, and problems such as timewise postoperative displacement can be prevented in advance.

### Brief Description of Drawings

[FIG. 1] FIG. 1 is a perspective view illustrating a spinous process spacer according to one embodiment of the present invention.
[FIG. 2] FIG. 2 is a plan view of the spinous process spacer of FIG. 1.
[FIG. 3] FIG. 3 is a side view of the spinous process spacer of FIG. 1.
[FIG. 4] FIG. 4 is a front view of the spinous process spacer of FIG. 1.
[FIG. 5] FIG. 5 is a schematic diagram for explaining the operation of the spinous process spacer of FIG. 1.
[FIG. 6] FIG. 6 is a perspective view illustrating a first modified example of the spinous process spacer of FIG. 1.
[FIG. 7] FIG. 7 is a perspective view illustrating a second modified example of the spinous process spacer of FIG. 1.
[FIG. 8] FIG. 8 is a perspective view illustrating a third modified example of the spinous process spacer of FIG. 1.
[FIG. 9] FIG. 9 is a diagram for explaining the operation when the spinous process spacers of FIG. 8 are rotated about their axes.
[FIG. 10A] FIG. 10A is a transverse cross-sectional view illustrating the cross-sectional shape of another modified example of the spinous process spacer of FIG. 1.
[FIG. 10B] FIG. 10B is a transverse cross-sectional view illustrating the cross-sectional shape of another modified example of the spinous process spacer of FIG. 1.
[FIG. 10C] FIG. 10C is a transverse cross-sectional view illustrating the cross-sectional shape of another modified example of the spinous process spacer of FIG. 1.
[FIG. 10D] FIG. 10D is a transverse cross-sectional view illustrating the cross-sectional shape of another modified example of the spinous process spacer of FIG. 1.
[FIG. 10E] FIG. 10E is a transverse cross-sectional view illustrating the cross-sectional shape of another modified example of the spinous process spacer of FIG. 1.
[FIG. 11] FIG. 11 is a perspective view illustrating another modified example of the spinous process spacer of FIG. 1.

### Explanation of Reference Signs:

1: spinous process spacer
2, 3: end face
5, 6: flat face (engaging portion)

### Best Mode for Carrying Out the Invention

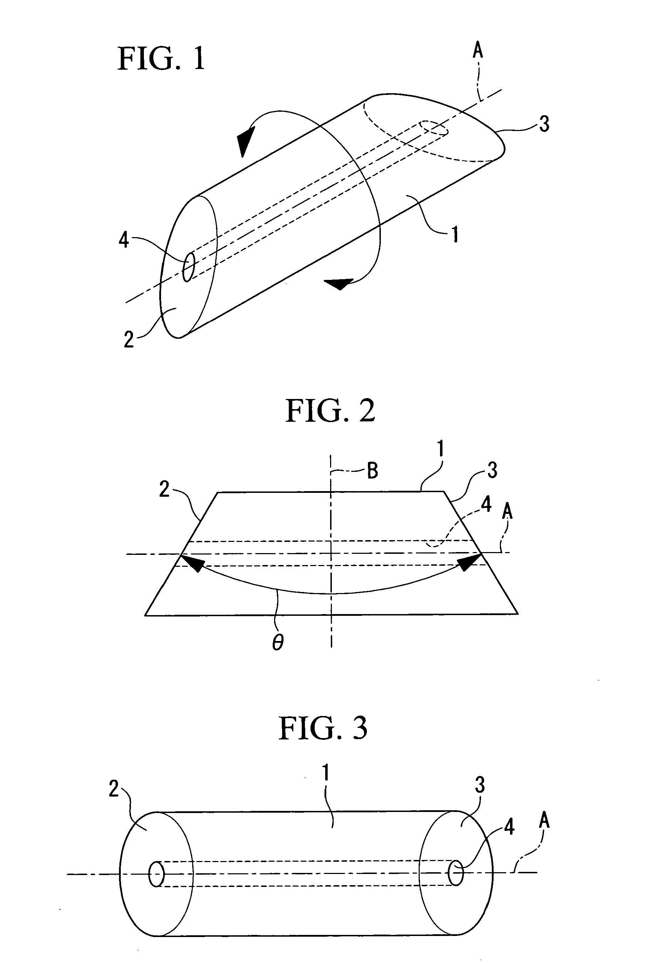

Hereunder is a description of a spinous process spacer 1 according to a first embodiment of the present invention, with reference to FIG. 1 to FIG. 5.
As shown in FIG. 1, the spinous process spacer 1 according to the present embodiment is made of cylindrical shaped calcium phosphate wherein opposite end faces 2 and 3 thereof are inclined with respect to the central axis A.

As shown in FIG. 2, the opposite end faces 2 and 3 are constituted by planes which are inclined respectively in reverse directions at a same angle with respect to the central axis A. Therefore, the spinous process spacer 1 according to the present embodiment has mirror symmetry about a plane B which passes through the longitudinal center and is orthogonal to the central axis A. The angle θ formed by these two end faces 2 and 3 is set at an angle selected from a range of not smaller than 30° and not larger than 90°, for example.

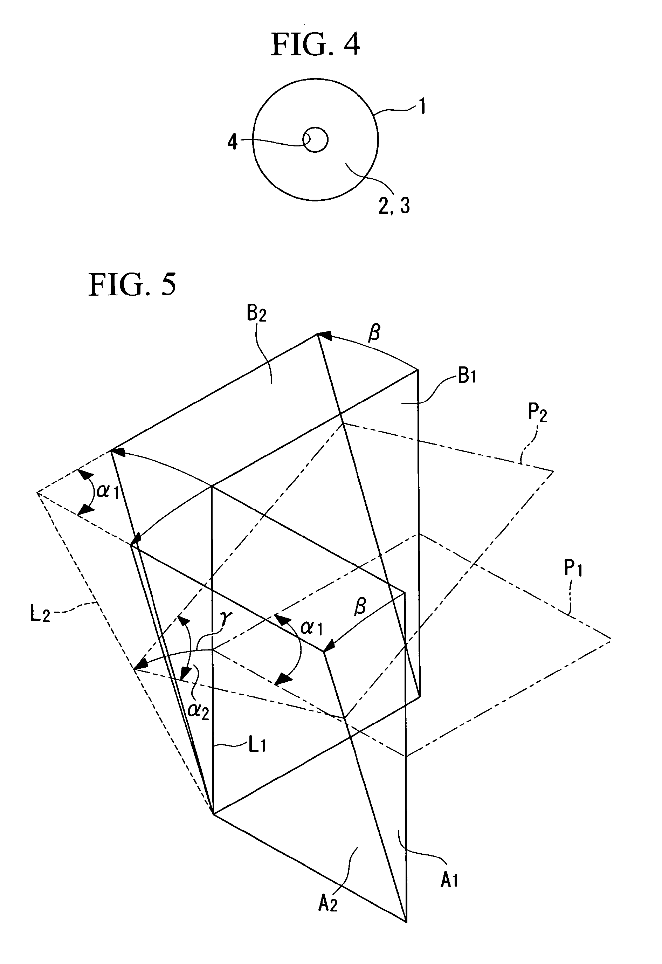

As a result, the spinous process spacer 1 according to the present embodiment has a trapezoid-shaped cross section outspreading to one side when viewed from the direction of FIG. 2, but has non-trapezoid-shaped cross sections when viewed from other directions as shown in FIG. 3 and FIG. 4.
Moreover, the spinous process spacer 1 according to the present embodiment is provided with a through hole 4 which extends in the longitudinal direction and has ports at the opposite end faces 2 and 3. The through hole 4 is designed to be used in, for example, a case where the spinous process spacer 1 inserted between split halves of the spinous process is to be sutured to the split spinous process.

Hereunder is a description of the operation of the spinous process spacer 1 according to the present embodiment constituted in such a manner.
As shown in the reference signs A₁ and B₁ in FIG. 5, when split faces of the split spinous process are opened to have an open angle α₁ only in the horizontal direction so as to insert the spinous process spacer 1 therebetween, the split faces A₁ and B₁ intersect each other in the cross line L₁ to have a horizontal reference plane P₁.

Subsequently from this situation, when the split faces are also opened in the vertical direction to have an open angle β, the split faces are arranged as shown in the reference signs A₂ and B₂. The split faces A₂ and B₂ intersect each other in the cross line L₂ that is located on their extensions. The cross line L₂ is inclined at an angle γ with respect to the cross line L₁, and a reference plane P₂ orthogonal thereto is also rotated at the same angle γ.

In this manner, the rotation of the reference plane from P₁ to P₂ due to the movement of the vertical open angle β is performed only in one direction. Therefore, by rotating the spinous process spacer 1 according to the present embodiment about its central axis A, the inclination directions of the opposite end faces 2 and 3 of the spinous process spacer 1 can be readily aligned with directions along the reference planes P₁ and P₂ that are the inclination directions of the split faces.

The angle α₂ formed by the split faces A₂ and B₂ is defined as an angle in the reference plane P₂. Therefore, as shown in FIG. 5, the angle α₂ is larger than the open angle α₁ formed when the split faces are opened only in the horizontal direction.
In the spinous process spacer 1 according to the present embodiment, the angle formed by the opposite end faces 2 and 3 is fixed at θ=α₁. Therefore, if the angle α₂ formed by the split faces A₂ and B₂ remains larger than the angle α₁, wedge-shaped gaps are formed respectively between the opposite end faces 2 and 3 and the split faces A₂ and B₂. Therefore, by reducing the horizontal open angle α₂ itself by the amount incremented due to the rotation of the reference plane from P₁ to P₂, the angle θ formed by the opposite end faces 2 and 3 of the spinous process spacer 1 can be aligned with the angle α₂ formed by the split faces A₂ and B₂.

In reality, for opening the interspace between split halves of the split spinous process, the interspace is pressingly opened against the elastic restoring force of the split spinous process, and inserted with the spinous process spacer 1. Therefore, if the inclination direction of the spinous process spacer 1 is aligned with the reference plane P₂ of the split faces A₂ and B₂ by rotating the spacer about its central axis A, then the split spinous process is automatically pushed back to directions reducing the open angle α₂ due to the elastic restoring force, and is eventually settled down in a position where the split faces A₂ and B₂ are closely attached to the end faces 2 and 3.

In this manner, according to the spinous process spacer 1 of the present embodiment, even if the vertical open angle β varies due to individual difference, the opposite end faces 2 and 3 of the spinous process spacer 1 can be closely attached to the split face A₂ and B₂ of the split spinous process in a gapless manner by the following manner. By rotating the spinous process spacer 1 about its central axis A, the inclination directions of the opposite end faces 2 and 3 are aligned with the reference plane P₂ of the split faces A₂ and B₂. Then, the horizontal open angle α₂ formed by the split faces A₂ and B₂ is adjusted.
Therefore, according to the spinous process spacer 1 of the present embodiment, the spinous process spacer 1 can be stably inserted and fixed between split halves of the spinous process, and timewise displacement can be prevented in advance.

Moreover, according to the spinous process spacer 1 of the present embodiment, there is no need of made-to-order production of the spinous process spacer 1 for each patient, and the same spinous process spacer 1 can be applied to every patient. As a result, advantages are provided in that mass production becomes possible to achieve cost reduction, and an extremely simple shape can be employed to considerably reduce the production cost.

Moreover, the spinous process spacer 1 according to the present embodiment is formed in a cylindrical shape. Therefore, if it is rotated about its central axis A, the position of the outer peripheral surface is hardly changed. Accordingly, even if the spinous process spacer 1 is rotated about its central axis A so as to manipulate the vertical open angle β which varies due to individual difference, the outer peripheral surface thereof is not protruded outwardly from the split spinous process. Also, there is no possibility of contacting with the spinal cord. Accordingly, advantages are provided in that the spinous process spacer 1 can be prevented from interfering with an adjacent spinous process or another spinous process spacer 1 fixed to the concerned spinous process.

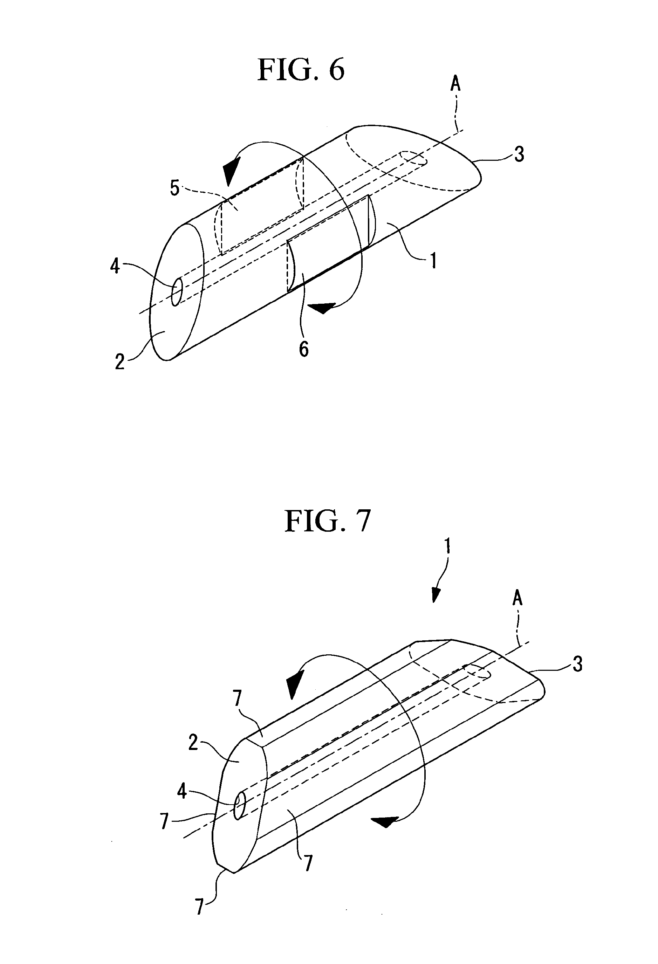

The spinous process spacer 1 according to the present embodiment is formed in a simple and approximately cylindrical shape; however, instead of this, the spacer may also be formed as shown in FIG. 6 in which the outer peripheral surface is provided with two parallel flat faces 5 and 6 extending along the central axis A. By so doing, the spinous process spacer 1 can be readily rotated about its central axis A by clipping the spacer at these two flat faces 5 and 6 with a general-purpose tool such as a spanner (not shown).
Moreover, the engaging portion to be provided on the outer peripheral surface of the spinous process spacer 1 is not limited to the two flat faces 5 and 6, and any form that can be engaged by a general-purpose or special tool may be employed.

Moreover, the spinous process spacer 1 according to the present embodiment is formed in a simple and approximately cylindrical shape; however, instead of this, the spacer may also be formed in a column shape as shown in FIG. 7 which has a plurality of planes 7 all over the entire longitudinal length. Moreover, the spinous process spacer 1 is not limited to such a cylindrical shape but may be formed in a polygonal prism shape, as long as the outer peripheral surface is not protruded outwardly from the split faces of the split spinous process due to the rotation about the central axis A.

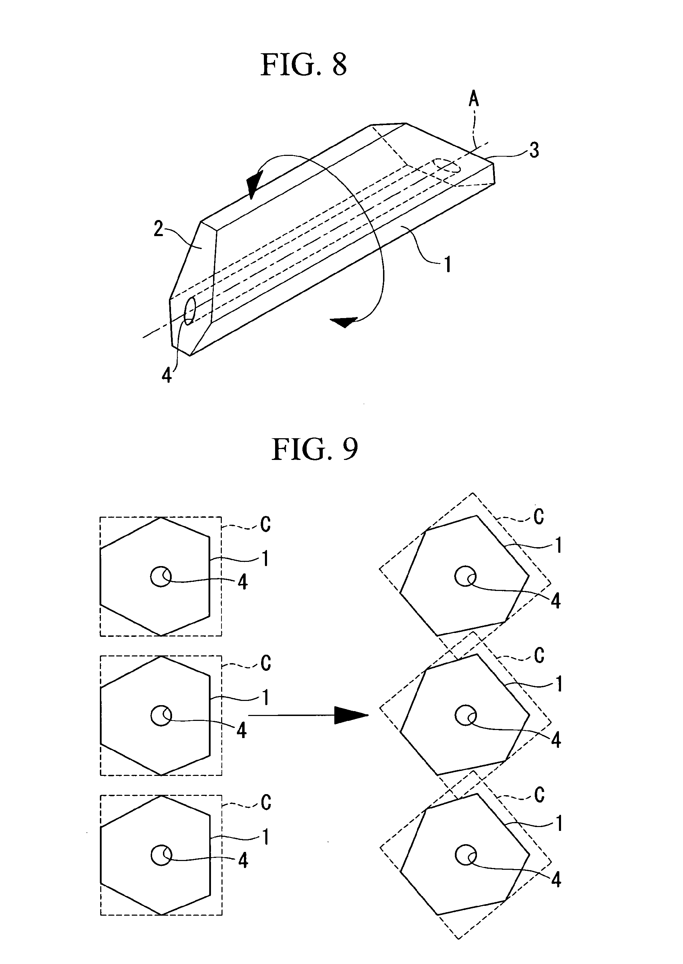

For example, as shown in FIG. 8 and FIG. 9, a spinous process spacer 1 formed in a polygonal prism shape having five or more radially outward convex angles may also be employed as the approximately cylindrical-shaped spinous process spacer 1. In examples shown in FIG. 8 and FIG. 9, spinous process spacers 1 formed in hexagonal prism shapes are illustrated; however, the spinous process spacer 1 is not limited to these but may be formed in a pentagonal prism shape, a heptagonal prism shape, or any other polygonal prism shape having more angles.

By so doing, even if the spinous processes spacer 1 is rotated in one direction for closely attaching the opposite end faces 2 and 3 of the spinous process spacer 1 to the split faces A₂ and B₂ of the split spinous process in a gapless manner, enough spaces can be held to avoid interference with adjacent spinous process spacers 1. In FIG. 9, the broken lines C denote cross-sectional shapes, in a case of a square prism shape, where adjacent spinous process spacers are in contact with each other due to rotation.

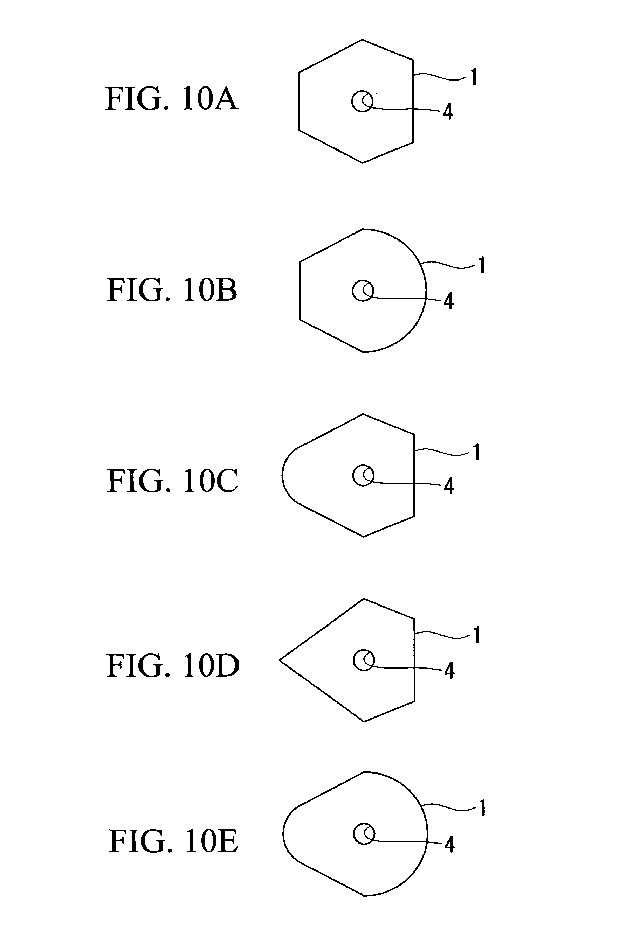

Moreover, in order to manipulate the vertical open angle β, the spinous process spacer 1 may also have a prismatic shape or a peripheral shape, whose cross-sectional shape will not allow protrusion of the outer peripheral surface(s) limitedly within a range of the rotation angle for rotating the spinous process spacer 1 about the central axis A.
For example, as shown in FIG. 10A to FIG. 10E, any approximately cylindrical-shaped spinous process spacer 1 having an optional cross-sectional shape including cylindrical surface(s) and one or more angle(s) may also be employed.

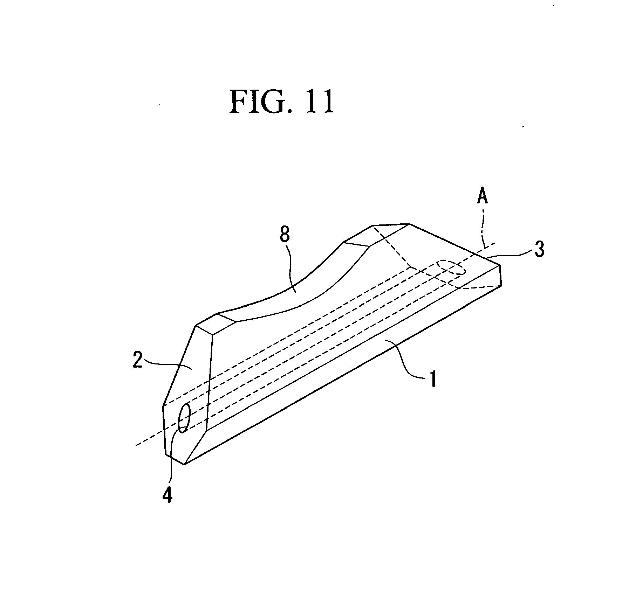

Moreover, the through hole 4 for suture is provided only in one locus along the central axis A, although it is needless to say that such holes may also be provided in a plurality of loci.
Furthermore, the locus of the through hole 4 may be deviated from the central axis of the spinous process spacer 1; in which case, as shown in FIG. 11, a cut 8 may also be partially formed in a portion concerned to be protruded in one direction to interfere with the spinal cord or other sites, if such interference is conceivable.

## Claims

1. A spinous process spacer for insertion between split halves of a spinous process resulting from longitudinal splitting of the spinous process, wherein the spinous process spacer is formed in an approximately cylindrical shape and the opposite end faces thereof are constituted by planes which mutually form a fixed angle.

2. A spinous process spacer according to claim 1, wherein the spinous process spacer has an approximately mirror symmetric shape about a plane which passes through the longitudinal center and is orthogonal to the central axis.

3. A spinous process spacer according to claim 1, wherein the outer peripheral surface is provided with an engaging portion to be engaged with a tool for applying a rotational force about the axis.

4. A spinous process spacer according to claim 3, wherein said engaging portion is formed of two mutually-parallel flat faces extending along the axis.

5. A spinous process spacer according to claim 1, wherein said opposite end faces are provided at an angle not smaller than 30° and not larger than 90°.

6. A spinous process spacer according to claim 1, wherein the spinous process space is made of a biocompatible ceramic material.
